# FASCICULE DE BREVET EUROPEEN

(11) **EP 1 231 908 B1**
(45) Date de publication et mention de la délivrance du brevet: **11.02.2004**
(21) Numéro de dépôt: 00951577.6
(22) Date de dépôt: 09.06.2000
(51) Int. Cl.: A61K 31/04, A61K 31/195, A61K 31/535, A61K 33/26, A61K 33/24, A61K 31/26, A61P 7/06, A61P 21/00

(54) **COMPOSITION PHARMACEUTIQUE COMPRENANT UN COMPOSE DONNEUR DE NO ET SON UTILISATION EN THERAPIE**
PHARMAZEUTISCHE ZUSAMMENSETZUNG DIE EINEN NO DONOR ENTHÄLT UND DESSEN THERAPEUTISCHE ANWENDUNG
PHARMACEUTICAL COMPOSITION COMPRISING A NO DONOR COMPOUND AND ITS USE

(30) Priorité: 11.06.1999 FR 9907442
(43) Date de publication de la demande: 21.08.2002
(73) Titulaire: CENTRE NATIONAL DE LA RECHERCHE SCIENTIFIQUE (CNRS), 75794 Paris Cedex 16 (FR)
(72) Inventeur: ISRAEL, Maurice, F-91440 Bures-sur-Yvette (FR); DE LA PORTE, Sabine, F-78000 Versailles (FR); FOSSIER, Philippe, F-95380 Louvres (FR); CHAUBOURT, Emmanuel, F-16330 Vars (FR); BAUX, Gérard, F-91700 Sainte Geneviève des Bois (FR); LEPRINCE, Christiane, F-91190 Gif-sur-Yvette (FR)
(74) Mandataire: Ahner, Francis
(86) Numéro de dépôt international: PCT/FR2000/001612
(87) Numéro de publication internationale: WO 2000/076451

(56) Documents cités:
- EP-A- 0 870 763
- WO-A-95/28377
- WO-A-96/02241
- WO-A-97/25984
- WO-A-97/33173
- WO-A-98/13358
- WO-A-99/01427
- DE-U- 29 709 820
- US-A- 5 885 621
- YAMADA, SHUICHI: "Effects of ATP and amino acid mixtures on progressive muscular dystrophy" SAPPORO IGAKU ZASSHI (1965), 27, 284-97, XP002131218
- BREDT D.S.: "Endogenous nitric oxide synthesis: Biological functions and pathophysiology." FREE RADICAL RESEARCH, (1999) 31/6 (577-596)., XP002131219
- HAYCOCK J W ET AL: "Oxidative damage to muscle protein in Duchenne muscular dystrophy." NEUROREPORT, (1996 DEC 20) 8 (1) 357-61., XP000879014
- CHAO, DANIEL S. ET AL: "Selective loss of sarcolemmal nitric oxide synthase in Becker muscular dystrophy" J. EXP. MED. (1996), 184(2), 609-618, XP000879016
- LAINE, ROMUALD ET AL: "Neuronal nitric oxide synthase isoforms.alpha. and.mu. are closely related calpain-sensitive proteins" MOL. PHARMACOL. (1998), 54(2), 305-312, XP000879162
- DECRORY, A. ET AL: "Mini- and full-length dystrophin gene transfer induces the recovery of nitric oxide synthase at the sarcolemma of mdx4cv skeletal muscle fibers" GENE THER. (1998), 5(1), 59-64, XP000879023
- AZZENA G B ET AL: "Nitric oxide regenerates the normal colonic peristaltic activity in mdx dystrophic mouse." NEUROSCIENCE LETTERS, (1999 FEB 12) 261 (1-2) 9-12., XP000879028
- THOMAS G.D. ET AL: "Impaired metabolic modulation of.alpha.-adrenergic vasoconstriction in dystrophin -deficient skeletal muscle." PROCEEDINGS OF THE NATIONAL ACADEMY OF SCIENCES OF THE UNITED STATES OF AMERICA, (1998) 95/25 (15090-15095)., XP002131220
- CHAUBOURT E. ET AL: "Nitric oxide and L-arginine cause an accumulation of utrophin at the sarcolemma: A possible compensation for dystrophin loss in Duchenne muscular dystrophy." NEUROBIOLOGY OF DISEASE, (1999) 6/6 (499-507)., XP002131221
- WAUGH, WILLIAM H. ET AL: "Evidence that L-arginine is a key amino acid in sickle cell anemia - a preliminary report" NUTR. RES. (N. Y.) (1999), 19(4), 501-518, XP000972387
- SCHECHTER A.N.: "NO Therapy." JOURNAL OF CLINICAL INVESTIGATION, (1997) 100/5 (955-956)., XP001028163
- HEAD, C. ALVIN ET AL: "Low concentrations of nitric oxide increase oxygen affinity of sickle erythrocytes in vitro and in vivo" J. CLIN. INVEST. ( 1997 ), 100(5), 1193-1198, XP001028165
- PERRINE S P ET AL: "BUTYRATE-INDUCED REACTIVATION OF THE FETAL GLOBIN GENES: A MOLECULAR TREATMENT FOR THE -HEMOGLOBINOPATHIES" EXPERIENTIA, BIRKHAUSER VERLAG. BASEL, CH, vol. 49, no. 2, 15 février 1993 (1993-02-15), pages 133-137, XP000564590 ISSN: 0014-4754
- PACELLI R. ET AL: "Hydroxyurea reacts with heme proteins to generate nitric oxide [6]." LANCET, (1996) 347/9005 (900)., XP001028057
- MORRIS, C. ET AL: "Effects of L-arginine therapy on nitric oxide production in patients with sickle cell disease." BLOOD, (NOV. 15, 1998) VOL. 92, NO. 10 SUPPL. 1 PART 1-2, PP. 695A. MEETING INFO.: 40TH ANNUAL MEETING OF THE AMERICAN SOCIETY OF HEMATOLOGY MIAMI BEACH, FLORIDA, USA DECEMBER 4-8, 1998 THE AMERICAN SOCIETY OF HEAMATOLOGY., XP001028060
- ENWONWU, C. O.: "Increased metabolic demand for arginine in sickle cell anemia" MED. SCI. RES. (1989), 17(23), 997-8, XP001028048
- SHER G.D. ET AL: "Extended therapy with intravenous arginine butyrate in patients with.beta.- hemoglobinopathies." NEW ENGLAND JOURNAL OF MEDICINE, (1995) 332/24 (1606-1610)., XP001028051
- MORRIS, CLAUDIA R. (1) ET AL: "L-arginine therapy paradoxically decreases nitric oxide production in patients with sickle cell disease." PEDIATRIC RESEARCH, (APRIL, 1999) VOL. 45, NO. 4 PART 2, PP. 150A. MEETING INFO.: ANNUAL MEETING OF THE AMERICAN PEDIATRIC SOCIETY AND THE SOCIETY FOR PEDIATRIC RESEARCH SAN FRANCISCO, CALIFORNIA, USA MAY 1-4, 1999, XP001028052
- ATZ A M ET AL: "Inhaled nitric oxide in sickle cell disease with acute chest syndrome." ANESTHESIOLOGY, (1997 OCT) 87 (4) 988-90., XP001028046

## Description

L'invention a pour objet l'utilisation d'au moins un composé capable de libérer ou d'induire la formation de NO dans les cellules, comme un donneur de NO ou un substrat de la NO synthase, pour ré-exprimer une protéine foetale dont l'isoforme adulte est mutée et/ou absente. L'invention trouve donc son intérêt dans le traitement des maladies où un gène adulte est défectueux ou absent. A titre d'exemple, l'utrophine, la forme homologue foetale de la dystrophine peut remplacer cette dernière dans les myopathies de Duchenne et de Becker. De même l'hémoglobine foetale peut remplacer l'hémoglogine adulte dans les cas de thalassémie et de drépanocytose. La présente invention est donc remarquable en ce qu'elle permet de remplacer les méthodes de traitement de ces pathologies proposées à ce jour par la voie du NO pour activer l'expression de la protéine foetale. L'invention se rapporte donc tout particulièrement à l'utilisation d'un donneur de NO ou d'un composé capable de libérer ou d'induire la formation de NO, selectionné parmi la L-arginine et ses dérivés dans les cellules pour la préparation d'un médicament destiné au traitement ou à la prévention des myopathies de Duchenne et Becker.

Les travaux réalisés sur la drépanocytose et la thalassémie ont mis en évidence que l'hydroxy-urée et le butyrate sont capables de réactiver l'expression du gène foetal de l'hémoglobine. Ce résultat pourrait être expliqué par des phénomènes métaboliques communs. Le cycle de l'urée et le cycle de Krebs sont couplés et si l'hydroxy-urée interfère avec le cycle de l'urée, elle pourrait conduire à une rétro-régulation du cycle de Krebs, ce qui entraînerait une plus faible consommation de l'acétyl-CoA et ainsi la formation de corps cétoniques comme le bêta-hydroxybutyrate.

Les phénomènes métaboliques associés à l'expression de gènes foetaux correspondent à un faible métabolisme oxydatif et à une forte glycolyse. Ainsi, l'analyse histochimique de fibres musculaires foetales a montré que ce sont davantage les enzymes glycolytique que les enzymes oxydatives qui sont exprimées. En outre, il a été montré que le mode de sécrétion de l'azote chez l'embryon est plutôt ammonotélique que ureotélique ce qui correspond à un fonctionnement ralenti du cycle de l'urée. Dans ces conditions, la L-arginine, qui est un substrat essentiel du cycle de l'urée, est détournée vers d'autres voies comme celles de la NO-synthase (NOS) ou de l'amidinotransférase, conduisant ainsi a une augmentation des niveaux d'oxyde nitrique (NO) et de créatine chez l'embryon.

La compréhension de ces phénomènes métaboliques a conduit les inventeurs à reproduire cette situation métabolique chez des animaux adultes et dans des systèmes de cellules en culture et ainsi de montrer que l'utilisation de L-arginine permettait de réactiver l'expression de gènes foetaux dans des tissus adultes de façon à restaurer la présence et la localisation de protéines foetales.

Les travaux ayant conduit à la présente invention ont été réalisés dans le but de traiter, par cette nouvelle stratégie de réactivation d'un gène foetal, des malades atteints de myopathies de Duchenne et Becker, mais la compréhension des phénomènes métaboliques rapportées ci-dessus permettent de les transposer aux traitement de toute maladie où le gène adulte défectueux a un homologue foetal.

La dystrophie musculaire de Duchenne, ci-après désignée DMD, est une maladie génétique lié au chromosome X dans laquelle on observe un manque d'une protéine du cytosquelette membranaire, la dystrophine, conduisant à une dégénérescence musculaire progressive. Trois types de traitement de la DMD sont envisagés aujourd'hui, un traitement pharmacologique avec des glucocorticoïdes, la transplantation de myoblastes et la thérapie génique (10). Il a aussi été proposé de compenser la perte de dystrophine en réactivant l'expresion de l'utrophine. En effet, il semble que l'utrophine soit capable de réaliser les mêmes fonctions cellulaires que la dystrophine et puisse ainsi compenser l'absence de dystrophine (3, 7). L'utrophine est observée dans les muscles à la fois chez les patients atteints de DMD et chez les témoins (24). Bien que chez l'adulte le gène de l'utrophine ne soit pas totalement éteint, l'utrophine est considérée comme l'homologue foetal de la dystrophine. Ce qui change chez l'adulte est sa localisation : elle n'est plus trouvée dans le sarcolemme, où elle est remplacée par la dystrophine, mais elle persiste dans les cellules satellites, les jonctions neuromusculaires et les capillaires (20) où la NO-synthase (NOS) est particulièrement abondante. Parmi les différentes isoformes de la NOS, il existe une forme spécifique du muscle, la NOS-mu, qui est une isoforme issue d'un épissage alternatif présentant une activité catalytique équivalente à celle de l'isoforme neuronale (34). La NOS a été observée dans le sarcolemme à la fois des fibres à contraction rapide et lente (17, 31). Dans le cas des souris mdx, un modèle animal de la DMD, la NOS n'est pas ancrée dans le sarcolemme mais délocalisée à l'intérieur des fibres musculaires (5). Il a en outre été montré récemment que la localisation de la NOS a été restaurée après transfection du gène de la dystrophine dans les muscles des souris mdx (9). Ce qui suggère la participation de cette enzyme ou de son produit dans l'assemblage du complexe protéique présent sous le sarcolemme. Compte-tenu de ces observations, les Inventeurs ont mis en évidence la possibilité d'utiliser le NO pour ré-exprimer l'utrophine, l'hémoglobine foetale ou d'autres protéines foetales. On a proposé dans l'art antérieur, l'emploi de vasodilatateurs, comme des bains chauds, mais l'effet du NO ou d'un composé donneur de NO sur la ré-expression de l'utrophine et de l'hémoglobine foetale n'a jamais été décrit.

Les travaux réalisés dans le cadre de la présente invention ont montré que dans des myotubes en culture, la L-arginine et les composés donneurs de NO augmentent à la fois le niveau et la localisation membranaire d'utrophine. Après injection de L-arginine dans les muscles, la localisation d'utrophine au niveau de la membrane de la fibre musculaire apparaît chez les souris témoins et augmente chez les souris mdx (qui présentent une faible sur-expression naturelle).

Les mécanismes qui conduisent à l'expression et la localisation de l'utrophine au niveau du sarcolemme ne sont pas clairs. Le NO pourrait être capable de nitrer les tyrosines de certains facteurs de transcription qui sont normalement phosphorylés, favorisant ainsi l'expression de l'utrophine dans les myotubes et son adressage vers la membrane. Une autre explication pourrait être que le NO agit via la production de GMPc comme suggéré par la réduction de son action en présence d'OQD, un inhibiteur sélectif de la guanylate cyclase. Le produit de dégradation de la L-arginine pourrait ainsi contrôler l'organisation complexe des protéines sous la membrane de la fibre musculaire.

L'ARNm de l'utrophine dans le muscle a été observé tout au long du sarcolemme, avec une expression préférentielle au niveau de la jonction neuromusculaire (14, 40). Jusqu'à présent, deux molécules exprimées à la jonction neuromusculaire, l'agrine et l'hereguline neurales, ont été identifiées comme capable d'augmenter respectivement l'expression d'utrophine dans le cytoplasme (15) et les niveaux d'ARNm dé l'utrophine (16). Mais la possibilité d'utiliser ces molécules dans le traitement de la DMD reste à démontrer.

Le but de la présente invention est donc d'offrir une nouvelle stratégie de traitement de maladies résultant de la déficience d'un gène adulte en restaurant l'activité d'un gène foetal homologue audit gène adulte.

Ce but est atteint grâce à l'utilisation d'un composé donneur de NO ou d'un composé capable de libérer, d'induire ou de favoriser la formation de NO dans les cellules pour la préparation d'un médicament destiné au traitement ou à la prévention d'une maladie résultant de la déficience d'un gène adulte chez un individu possédant un gène foetal homologue audit gène adulte grâce à la ré-expression du gène foetale homologue lorsqu'il existe.

On entend par composé capable de libérer ou d'induire la formation NO, des composés comme des donneurs de NO ou des composés capable de favoriser la formation de NO dans le cellules.

Plus particulièrement, l'invention vise l'utilisation d'un composé donneur de NO ou d'un composé capable de libérer, de favoriser ou d'induire la formation de NO dans les cellules pour la préparation d'un médicament destiné à réactiver l'expression d'au moins un gène foetal dans des tissus adultes de façon à restaurer la présence et/ou la localisation d'au moins une protéine foetale.

L'utilisation selon l'invention permet de réactiver la situation foetale en ré-exprimant la forme embryonique de la protéine codée par le gène défecteux.

Certains composés comme l'hydroxyurée ou le bêta-hydroxybutyrate sont toxiques ou mal tolérés, aussi l'invention concerne plus particulièrement, à titre de composé capable d'induire la formation de NO, la L-arginine, ou ses dérivés comme l'hydroxy-arginine ou ses dérivés borés, qui favorisent la production de NO ou la préservation du substrat. Dans une forme de mise en oeuvre préférée de l'invention, la L-arginine est administrée à raison de 200 mg/kg pendant 3 a 4 semaines

Mais l'invention concerne très largement, l'utilisation des donneurs de NO ou de composés impliqués dans des voies métaboliques permettant d'augmenter la production cellulaire de NO.

Il est connu que les dystrophies de Duchenne et de Becker sont liées à la délétion ou à la mutation d'un gène du chromosome X. Ainsi, la dystrophine est une protéine essentielle à la fonction musculaire, dont l'absence ou la mutation conduit à une dégénérescence du muscle. La maladie évolue graduellement au fur et à mesure que le muscle dégénère en raison de l'absence de dystrophine. La présente invention vise précisément à réactiver la protéine embryonnaire qu'est l'utrophine pour traiter ou prévenir la DMD. Les travaux réalisés dans le cadre de la présente invention ont montré que l'injection d'une composition pharmaceutique comprenant au moins un composé donneur de NO ou encore capable de libérer, de favoriser ou d'induire la formation de NO dans les cellules, permet d'induire l'apparition d'utrophine au niveau du sarcolemme de muscles dystrophique et normaux, *in vitro* sur des cultures de myotubes. De même in vivo, on observe que l'injection d'une telle composition chez la souris entraîne une expression importante d'utrophine au niveau du sarcolemme.

En conséquence, l'invention se rapporte tout particulièrement à l'utilisation d'au moins un composé donneur de NO ou d'un capable de libérer, de favoriser ou d'induire la formation de NO dans les cellules pour la préparation d'un médicament pour ré-exprimer la protéine foetale comme roue de secours de la protéine adulte déficiente. Tout particulièrement, la méthode de l'invention permet de réactiver l'expression de l'utrophine dans des tissus adultes de façon à restaurer la présence et la localisation de cette protéine au niveau du sarcolemme, de façon à ce que l'utrophine remplace la dystrophine lorsque celle-ci est absente.

L'invention concerne donc également une composition pharmaceutique comprenant au moins un composé donneur de NO ou encore capable de libérer, de favoriser ou d'induire la formation de NO dans les cellules, associé dans ladite composition avec un véhicule pharmaceutiquement acceptable, pour une administration per os, per cutannée, intraperitonéale, intraveineuse ou sous-cutanée.

D'autres avantages et caractéristiques de l'invention apparaîtront de la description qui suit rapportant les travaux réalisés dans le cadre de l'invention sur la DMD.

La DMD (11) la plus fréquente (1 garçon sur 3500) et la plus sévère myopathie, est caractérisée par une perte progressive de la force musculaire, conduisant finalement à une fibrose marquée et une infiltration graisseuse. Le gène de la DMD (25) couvre environ 2300 kb sur la bande p21 et la plupart des mutations de la DMD sont des délétions intragéniques, conduisant à l'absence de dystrophine, une protéine de 427 kD, dans le muscle des patients (18, 1). La dystrophine est une large protéine du cytosquelette localisée à la surface interne du sarcolemme du muscle normal. La dystrophine est associée à un complexe de glycoprotéines et de protéines de membrane respectivement dénommées DAGs, désignant le terme anglais "dystrophin-associated glycoproteins", et DAPs, désignant le terme anglais (dystrophin-associated proteins), qui sont considérablement réduites dans le muscle des patients atteints de DMD (2, 28). L'une des protéines, la syntrophine, est associée à la NOS via un domaine PDZ (4). Le complexe dystrophine - glycoprotéine lie le cytosquelette subsarcolemmal à la matrice extracellulaire. La dystrophine est impliquée dans le maintien de la structure morphologique et fonctionnelle de la fibre du muscle strié et dans l'homéostasie du calcium.

Un transcrit autosomal de 13 kb codé par un gène du bras long du chromosome 6 chez l'homme et le chromosome 10 chez la souris, a été identifié. Il code une protéine présentant plus de 80% d'homologie avec la dystrophine, dénommée l'utrophine, de 395 kD (23, 36). L'homologie entre la dystophine et l'utrophine s'étend sur toute leur longueur suggérant qu'elles dérivent d'un gène ancestral commun. L'utrophine, comme la dystrophine, se lie à l'actine par le domaine N-terminal et le domaine C-terminal est hautement conservé. L'utrophine est associée à un complexe des protéines sarcolemmales identiques ou au moins antigéniquement similaire à ceux de la dystrophine. Sa localisation est la même que celle du récepteur à l'acétylcholine, en haut des plis post-synaptiques. L'utrophine est peut être l'une des molécules du cytosquelette qui organise et stabilise le domaine cytoplasmique du récepteur de l'acétylcholine.

Les patients atteints de DMD et de dystrophie de Becker, (une forme moins sévère de DMD) et la souris mdx, conserve une certaine expression de l'utrophine au niveau du sarcolemme (35, 20, 21, 24) probablement pour compenser l'absence de dystrophine. Les méthodes pour post-réguler l'expression du gène de l'utrophine sont bénéfiques pour la fonction musculaire. Par exemple, l'utilisation de l'expression transgénique d'abord de l'utrophine tronquée puis l'utrophine complète chez la souris, a permis de démontrer que l'utrophine peut fonctionnellement remplacer la dystrophine (8, 38, 39) : la surexpression d'utrophine conduit à la restauration de tous les composants des DAGs, et la performance du muscle est augmentée. La surexpression de l'utrophine sauve la détérioration du diaphragme, le muscle le plus sévèrement atteint chez la souris mdx. Par ailleurs, les souris déficientes en utrophine présentent un phénotype de myopathie légère, comme les souris mdx déficientes en dystrophine, mais les souris déficientes à la fois en dystrophine et utrophine présentent une myopathie sévère des muscles du squelette et cardiaque (33). L'expression d'un transgène de l'utrophine tronquée dans le muscle de souris déficientes à la fois en dystrophine et utrophine protège du décès et du développement de tout phénotype clinique (30).

Au cours du développement, l'utrophine est trouvée à la surface membranaire des fibres immatures chez les embryons normaux et est progressivement remplacée par la dystrophine, exceptée à la jonction neuromusculaire où elle persiste (26). Ainsi, il est possible de considérer l'utrophine comme l'homologue foetal de la dystrophine (36). Plusieurs observations ont mis en lumière le mécanisme qui gouverne le passage du gène foetal au gène adulte. Les patients atteints de drépanocytose ou de thalassémie qui présentent un gène adulte de l'hémoglobine anormal ont été traités avec le butyrate ou l'hydoxy-urée, ce qui a réactivé le gène foetal de l'hémoglobine (32, 29, 27). Il est possible d'escompter chez le foetus un niveau élevé de glycolyse (12, 6) avec un passage préférentiel de l'acétyl-CoA vers les voies anaboliques. La faible phosphorylation oxydative devrait favoriser les voies de l'acétyl-CoA vers les corps cétoniques. L'accumulation conséquente de bêta-hydroxybutyrate pourrait alors induire l'expression des gènes foetaux. Comme le cycle de Krebs et le cycle de l'urée sont couplés, la faible phophorylation oxydative est corrélée avec la faible production d'urée, laquelle peut aussi être induite par un traitement à l'hydroxy-urée. Ceci pourrait résulter en des taux élevés de L-arginine, qui pourrait alors être utilisée comme substrat de la NOS et de l'amidinotransférase conduisant ainsi à la créatine. L'oxyde nitrique (NO) donnerait alors le signal de l'expression des gènes foetaux, qui serait alors responsable des hauts niveaux de créatine observés dans l'urine des patients atteints de DMD. Les mécanismes envisagés ci-dessus par les inventeurs, les ont conduits à tester les effets de la L-arginine et de composés donneurs de NO sur l'expression d'utrophine. Les inventeurs ont ainsi démontré que de façon remarquable chez les souris adultes normales et mdx traitées chroniquement avec de la L-arginine, qui est un substrat de la NOS, les taux d'utrophine musculaire augmentaient au niveau de la membrane tout au long du sarcolemme. Les expériences rapportées ci-après montrent que de façon surprenante le traitement par la L-arginine, des donneurs de NO, augmente les niveaux d'utrophine et sa localisation membranaire dans des cultures de myotubes normaux et mdx. Des résultats similaires ont été obtenus avec l'hydroxy-urée qui a servi de référence car il est connu que ce produit active l'hémoglobine foetale.

### I - Méthode.

### 1) Traitement des souris.

Trois souris adultes de 18 mois normales (lignée C57 BL/6) et trois souris mdx ont reçu quotidiennement une injection intrapéritonéale de 200 mg/kg de L-arginine pendant trois semaines. Deux autres groupes de trois souris adultes ont servi de contrôle et ont reçu quotidiennement une injection de sérum physiologique.

Les souris ont été sacrifiées par anesthésie à l'éther, le biceps femoris et les muscles semi-tendineux ont été rapidement disséqués à partir des membres postérieurs de chaque animal et congelés dans de l'azote liquide.

### 2) Culture cellulaire.

Des myotubes ont été obtenus à partir d'une lignée cellulaire normale (NXLT) et une lignée cellulaire mdx comme décrit par Liberona et al. (22) et les myotubes C2 comme décrit par Inestrosa et al. (19).

### 3) Immunofluorescence.

*In vivo.* Après fixation avec du méthanol froid (-20°C pendant 10 minutes), les coupes de 7 µm ont été incubées pendant deux heures avec un anticorps monoclonal spécifique de l'utrophine (NCL-DRP 2, Novacastra) (1/10 vol/vol) dans du PBS contenant 0,1% de saponine et 0,2% d'albumine bovine. Le second anticorps marqué à la fluoroscéine (N 1031, Amersham) a été dilué (1/4000 vol/vol) dans du PBS contenant 0,1% de saponine et incubé pendant une heure.

*In vitro*. Les cultures ont été traitées comme décrit précédemment à l'exception du second anticorps marqué à la fluoroscéine qui a été dilué à 1/100 vol/vol. La durée d'incubation a été de 2 heures pour le premier et le second anticorps.

### 4) Immunoblotting.

Les myotubes obtenus à partir des lignées NXLT, XLT et C2 ont été homogénéisés avec un Polytron (Kinematica) dans 10 mM Tris-HCl pH 6,8, 1% Triton X-100, 1% SDS, 0,5% deoxycholate de sodium sur glace. La quantité de protéines totales a été déterminée par le protocole du test protéique à l'acide bicinchoninique (BCA; Pierce). Des quantités équivalentes de protéine ont été séparées par SDS-Page sur un gel à 5%, puis électrotransférées sur une membrane de nitrocellulose (Schleicher & Schuell). Les membranes ont ensuite été incubées avec le même anticorps monoclonal dirigé contre l'utrophine, utilisé pour les techniques d'immunofluorescence (1/250 vol/vol). Les anticorps fixés ont été détectés avec un anticorps secondaire de chèvre anti-souris de Sanofi (1/5000 vol/vol) liés à la peroxydase de raifort et révélés par réaction de chimieluminescence (ECL, Amersham Pharmacie Biotech).

### II - Résultats.

Il sera fait référence dans les résultats qui suivent aux illustrations en annexe dans lesquels :

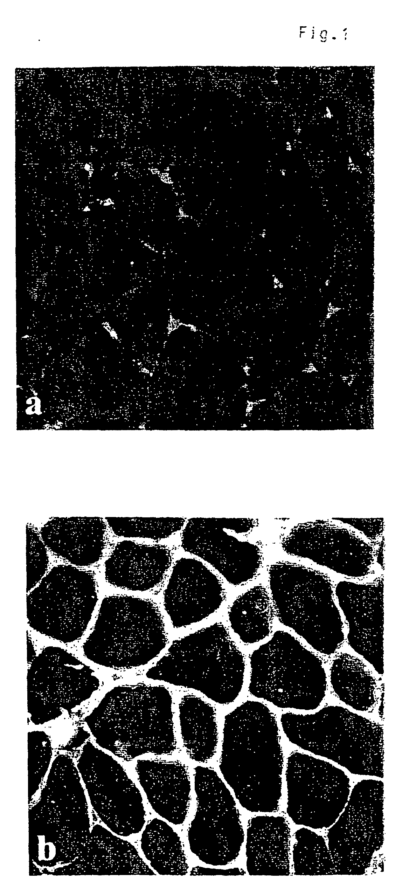

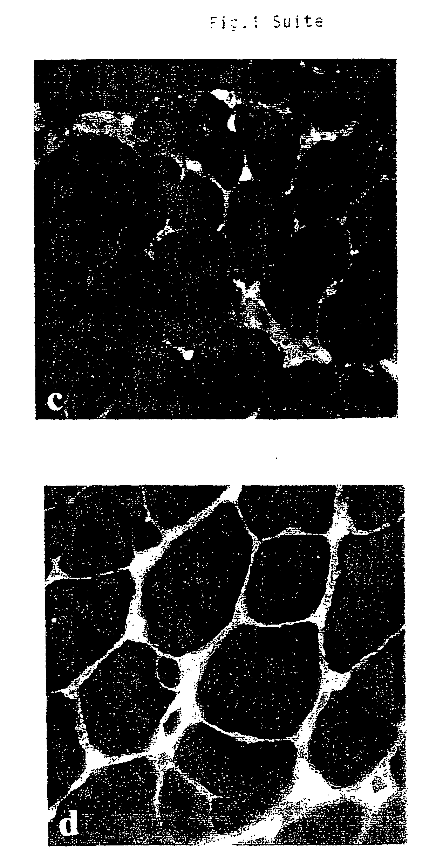

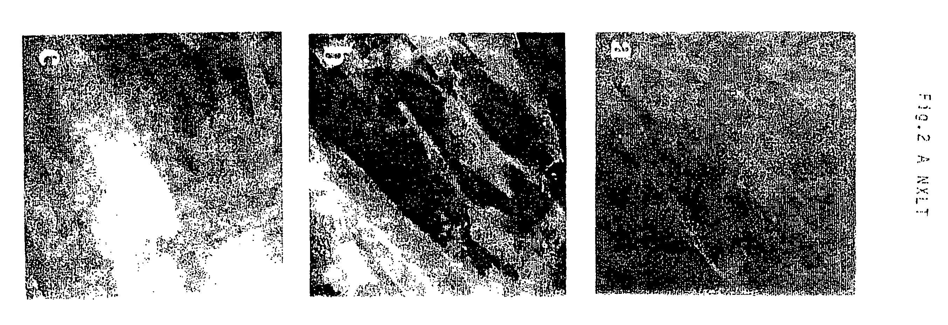

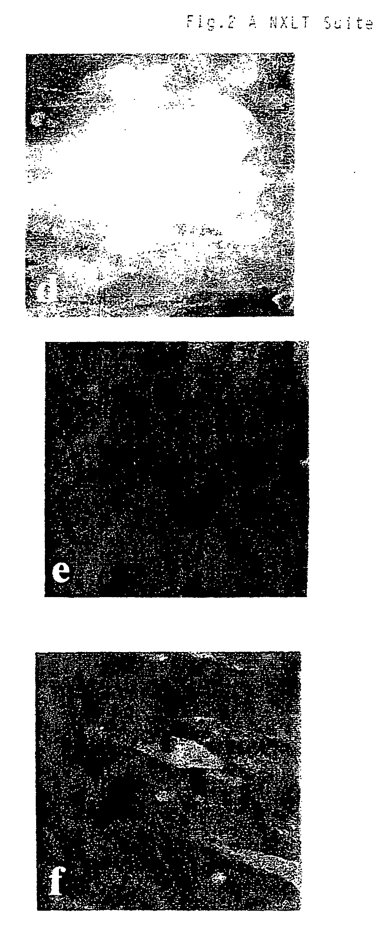

- La figure 1 représente l'apparition de l'utrophine sous le sarcolemme de souris adultes normales et mdx traitées chroniquement avec la L-arginine (grossissement X 300). La figure 1 montre l'immunolocalisation de l'utrophine sur la membrane des muscles des souris normales et mdx traitées à la L-arginine. (a) contrôle correspondant aux souris normales ayant reçu une injection de sérum physiologique ; pas d'utrophine observée au niveau du sarcolemme. (b) souris normales traitées à la L-arginine : on observe l'utrophine sous le sarcolemme. (c) contrôle correspondant au souris mdx ayant reçu une injection de sérum physiologique : de l'utrophine est visible au niveau du sarcolemme. (d) souris mdx ayant reçu de la L-arginine : augmentation des niveaux d'utrophine sous le sarcolemme.

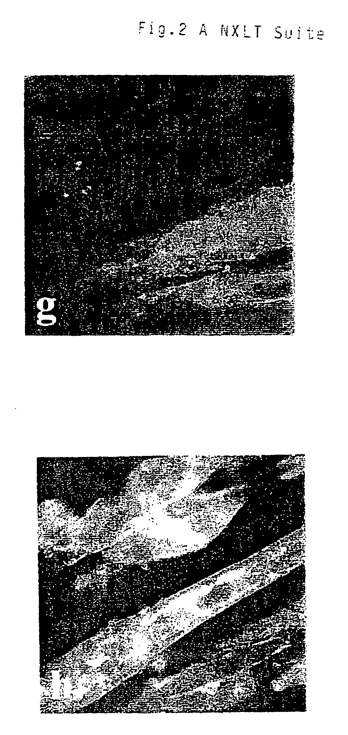

- La figure 2 représente la variation d'utrophine dans les myotubes après traitement impliquant l'oxyde nitrique (NO) (grossissement X 300). A, a-h : lignée cellulaire normale (NXTL). B, a'-h' : lignée cellulaire mdx (XLT). Les cultures cellulaires ont été traitées par exposition des myotubes différenciés aux drogues pendant 48 heures. A, a' : cultures contrôles. B, b' : L-arginine (2.10⁻³ M). c, c' : SIN-1 (10⁻³ M). d, d' : SIN-1 (10⁻³ M) + L-arginine (10⁻³ M).e, e' : D-arginine (10⁻³ M).f, f' : L-arginine (10⁻³ M) + OQD (10⁻⁵ M). g, g' : L-NMMA (10⁻³ M). h, h' : hydroxyurée (10⁻⁴ M).

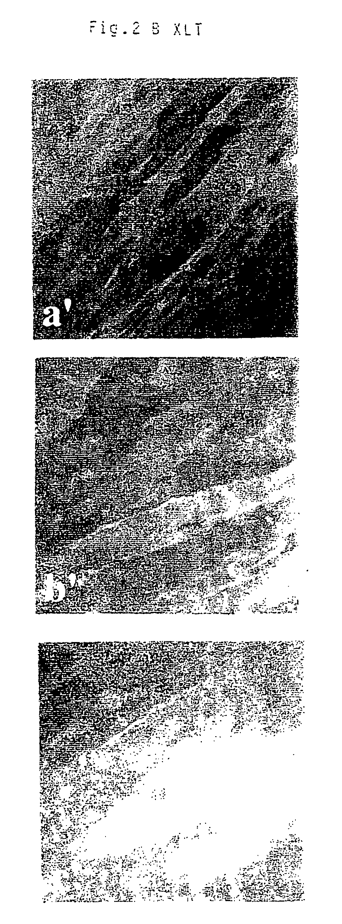

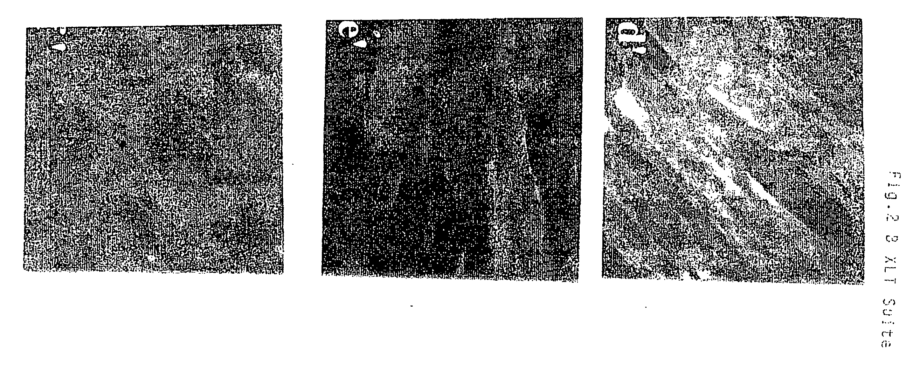

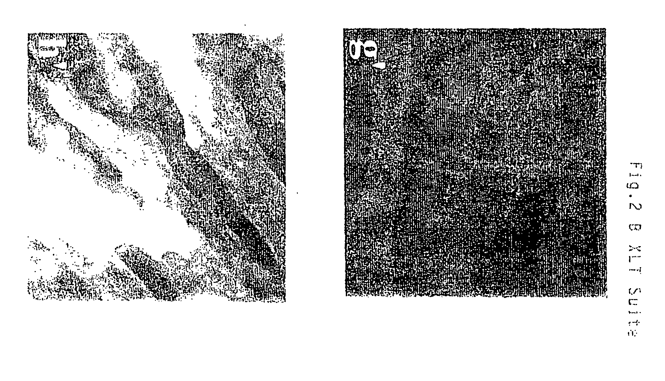

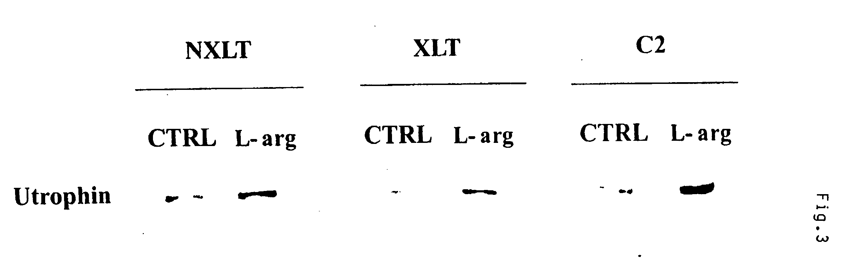

- La figure 3 représente l'augmentation des niveaux d'utrophine dans les myotubes NXLT, XLT et C2 par l'action de la L-arginine. Les analyses en immunoblot de l'utrophine ont été réalisées sous des conditions de contrôle (CTRL) et après 48 heures de traitement avec 2.10⁻³ M de L-arginine (L-arg).

Les souris adultes ayant reçu une injection intrapéritonéale de L-arginine pendant trois semaines ont été sacrifiées. Après sacrifice, les muscles de la cuisse ont été préparés par immunocytochimie. Après ce traitement, l'utrophine a été révélée au dessous du sarcolemme dans les fibres musculaires des souris normales comme montré sur la figure 1a. Le traitement avec la L-arginine des souris mdx a augmenté le niveau d'utrophine déjà présent dans le sarcolemme (35, 21). A la fois dans les souris normales et mdx, l'immunomarquage couvre le sarcolemme et est présent sur une partie du tissu interstitiel. Ce marquage est probablement du à l'utrophine exprimée par les capillaires et les cellules satellites.

Cet effet de l'arginine a ensuite été étudié sur des myotubes en culture qui sont plus adaptés pour une application directe des drogues et qui évitent l'interférence avec de l'utophine non musculaire. Les myotubes NXLT et XLT des souris normales et mdx, respectivement, ont été utilisés dans des tests immunochimiques des effets de la L-arginine et du NO sur l'expression d'utrophine. Après 48 heures de traitement, le marquage de l'utrophine a augmenté lorsque l'on a augmenté la synthèse de NO endogène via un excès de L-arginine et lorsque l'on a appliqué le SIN-1 comme montré sur la figure 2. L'utrophine a été co-localisée avec les larges amas de récepteurs à l'acétylcholine présents sur les myotubes attestant qu'une partie du marquage est membranaire (non illustré en annexe). L'augmentation du marquage de l'utrophine a été aussi observé à un plus faible degré sur les cellules de myotubes C2 de souris et les myotubes primaires de rat. L'application cumulée de SIN-1 et de L-arginine augmente encore le marquage de l'utrophine comme montré sur la figure 2. L'absence d'effet de la D-arginine rapporté sur la figure 2 démontre l'implication du NO dans la méthode de l'invention. Le niveau basal d'utrophine en l'absence d'activité de NO-synthétase de la figure 2 a été obtenu après application de N^{G}-méthyl-L-arginine (L-NMMA), qui est un inhibiteur des NOS. Il est largement admis que les effets intracellulaires du NO sont médiés à travers l'activation de la guanylate cyclase soluble .La synthèse d'utrophine induite par le NO a été inhibée en présence de ODQ (13), qui est un antagoniste spécifique de la guanylate cyclase comme montré sur la figure 2. La figure 2 montre également que l'hydroxy-urée utilisée par analogie avec le traitement de la thalassémie, augmente également de façon remarquable le marquage de l'utrophine. Cet effet résulte probablement d'une action sur l'expression de l'utrophine.

Afin de compléter l'analyse de l'effet de la production de NO sur l'expression d'utrophine chez les souris normales et mdx, les inventeurs ont extrait les protéines des cultures de myotubes traitées ou non avec la L-arginine dans les mêmes conditions que précédemment. Les western-blots de la figure 3 montrent une augmentation claire de l'utrophine dans les deux types de lignées cellulaires, confirmant ainsi les données immunocytochimiques. Cette augmentation en utrophine après traitement par la L-arginine a été confirmée dans une lignée cellulaire de myotubes C2 (figure 3).

### Références bibliographiques

1. Ahn, A. H., & Kunkel, L. M. (1993) The structural and functional diversity of dystrophin. *Nat. Genet*. **3,** 283-291.
2. Appel, E. D., Roberds, S. L., Campbell, K. P., & Merlie, J. P (1995) Rapsyn may function as a link between the acetylcholine receptor and the agrin-binding dystrophin-associated glycoprotein complex. *Neuron* **15,** 115-126.
3. Blake, D. J., Tinsley, J. M., & Davies, K. E. (1996) Utrophin: a structural and functional comparison to dystrophin. *Brain Pathol.* **1,** 37-47.
4. Brenman, J. E., Chao, D. S., Gee, S. H., McGee, A. W., Craven, S. E., Santillano, D. R., Wu, Z., Huang, F., Xia, H., Peters, M. F., Froehner, S. C., & Bredt, D. S. (1996). Interaction of nitric oxide synthase with the postsynaptic density protein PSD-95 and β1-syntrophin mediated by PDZ domains. *Cell* **84,** 757-767.
5. Brenman, J. E., Chao, D. S., Xia, H., Aldape, K., & Bredt, D. S. (1995) Nitric oxide synthase complexed with dystrophin and absent from skeletal sarcolemma in Duchenne muscular dystrophy. *Cell* **82**, 743-752.
6. Butler-Browne, G. S., Barbet, J. B., & Thornell, L. E. (1990) Myosin heavy and light chain expression during human skeletal muscle development and precocious muscle maturation induced by thyroid hormone. *Anat. Embryol*. (Berl.) **181,** 513-522.
7. Campbell, K. P., & Crosbie, R. H. (1996) Utrophin to the rescue. *Nature* **384,** 308-309.
8. Deconinck, N., Tinsley, J., DeBacker, F., Fisher, R., Kahn, D., Phelps, D., Davies, K., & Gillis, J.M. (1997) Expression of truncated utrophin leads to major functional improvements in dystrophin-deficient muscles of mice. *Nature Medecine* **3,** 1216-1221.
9. Decrouy, A., Renaud, J. M., Lunde, J. A., Dickson, G., & Jasmin, B. J. (1998) Mini- and full-length dystrophin gene transfer induces the recovery of nitric oxide synthase at the sarcolemma of mdx4cv skeletal muscle fibers. *Gene Ther*. **5,** 59-64.
10. De La Porte, S., Morin, S., & Koenig, J. (1999) Characteristics of skeletal muscle in mdx mutant mice. *Int. Rev. Cytol.* **191,** 99-148.
11. Engel, A. G., Yamamoto, M., & Fischbeck, K. H. (1994) Dystrophinopathies, in: Myology (McGraw-Hill, Inc.) **2**, 1133-1187.
12. Farkas-Bargeton, E., Diebler, M. F., Arsenio-Nunes, M. L., Wehrle, R., & Rosenberg, B. (1977) Histochemical, quantitative and ultrastructural maturation of human foetal muscle. *J. Neurol. Sci*. **31,** 245-259.
13. Garthwaite, J., Southam, E., Boulton, C. L., Nielsen, E. B., Schmidt, K., & Mayer, B. (1995) Potent and selective inhibition of nitric oxide-sensitive guanylyl cyclase by 1H-(1,2,4) oxadiazolo (4,3-a) quinoxalin-1-one. *Mol. Pharmacol*. **48,** 184- 188.
14. Gramolini, A. O., Dennis, C. L., Tinsley, J. M., Robertson, G.S., Cartaud, J., Davies, K. E. & Jasmin, B. J. (1997) Local transcriptional control of utrophin expression at the neuromuscular synapse. *J. Biol. Chem.* **272,** 8117-8120.
15. Gramolini, A. O., Burton, E. A., Tinsley, E.A., Ferns, M. J., Cartaud, A., Cartaud, J., Davies, K. E., Lunde, J. A., & Jasmin, B. J. (1998) Muscle and neuronal isoforms of agrin increase utrophin expression in cultured myotubes via transcriptional regulatory mechanisms. *J. Biol. Chem.* **273,** 736-743.
16. Gramolini, A. O., Angus, L. M., Schaeffer, L., Burton, E. A., Tinsley, J. M., Davies, K. E., Changeux, J. P., & Jasmin, B. J. (1999) Induction of utrophin gene expression by heregulin in skeletal muscle cells:: role of the N-box motif and GA binding protein. *Proc. Natl. Acad. USA* **96,** 3223-3227.
17. Grozdanovic, Z., Gosztonyi, G., & Gossrau, R. (1996) Nitric oxide synthase 1 (NOS-1) is deficient in the sarcolemma of striated muscle fibers in patients with Duchenne muscular dystrophy, suggesting an association with dystrophin. *Acta Histochem.* **98,** 61-69.
18. Hoffman, E. P., Brown, R H., & Kunkel, L. M. (1987) Dystrophin: the protein product of the Duchenne muscular dystrophy locus. *Cell* **51,** 919-928.
19. Inestrosa, N. C., Miller, J. B., Silberstein, L., Ziskind-Conhaim, L., & Hall, Z. W. (1983) Developmental regulation of 16S acetylcholinesterase and acetylcholine receptors in a mouse muscle cell line. *Exp. Cell Res.* **147,** 393-405.
20. Karpati, G., Carpenter, S., Morris, G. E., Davies, K. E., Guerin, C., & Holland, P. (1993) Localization and quantification of the chromosome 6-encoded dystrophin-related protein in normal and pathological human muscle. *J. Neuropath. Exp. Neurol.* **52,** 119-128.
21. Koga, R., Ishiura, S., Takemitsu, M., Kamakura, K., Matsuzaki, T., Arahata, K., Nonaka, I., & Sugita, H. (1993) Immunoblot analysis of dystrophin-related protein (DRP). *Biochim. Biophys. Acta* **1180**, 257-261.
22. Liberona, J. L., Powell, J. A., Shenoi, S., Petherbridge, L., Caviedes, R., & Jaimovich, E. (1998). Differences in both inositol 1, 4, 5,-triphosphate mass and inositol 1, 4, 5-triphospate receptors between normal and dystrophic skeletal muscle cell lines. *Muscle* & Nerve 21, 902-909.
23. Love, D. R., Hill, D. F., Dickson, G., Spurr, N. K., Byth, B. C., Marsden, R. F., Walsh, F. S., Edwards, Y. H., & Davies, K. E. (1989) An autosomal transcript in skeletal muscle with homology to dystrophin. *Nature* **339**, 55-58.
24. Mizuno, Y., Nonaka, I., Hirai, I., & Ozawa, E. (1993) Reciprocal expression of dystrophin and utrophin in muscles of Duchenne muscular dystrophy patients, female DMD-carriers and control subjects. *J. Neurol. Sci.* **119,** 43-52.
25. Monaco, A. P., Neve, R. L., Colleti-Feener, C., Bertelson, C. J., Kurnit, D. M., & Kundel, L. M. (1986) Isolation of candidate cDNAs for portions of the Duchenne muscular dystrophy gene. Nature (London) **323,** 646-650.
26. Oliver, L., Goureau, O., Courtois, Y., & Vigny, M. (1996) Accumulation of NO synthase (type-1) at the neuromuscular junctions in adult mice. *NeuroReport* **7**, 924-926.
27. Olivieri, N. F., & Weatherall, D. J. (1998) The therapeutic reactivation of foetal haemoglobin. *Hum. Mol. Genet.* **7,** 1655-1658.
28. Ozawa, E., Yoshida, M., Suzuki, A., Mizuno, Y., Hagiwara, Y., & Noguchi, S. (1995) Dystrophin-associated proteins in muscular dystrophy. *Hum. Molec. Genet.* **4,** 11711-11716.
29. Perrine, S. P., Ginder, G. D., Faller, D. F., Dover, G. H., Ikuta, T., Witkowska, E., Cai, S. P., Vichinsky, E. P., & Olivieri, N. F. (1993) A short-term trial of butyrate to stimulate fetal-globin-gene expression in the β-globin disorders. *N. Engl. J. Med.* **328,** 81-86.
30. Rafael, J. A., Tinsley, J. M., Potter, A. C., Deconinck, A. E. & Davies, K. E. (1998) Skeletal muscle-expression of a utrophin transgene rescues utrophindystrophin deficient mice. *Nature Genetics* **19,** 79-82.
31. Ribera, J., Marsal, J., Casanovas, A., Hukkanen, M., Tarabal, O., & Esquerda, J. E. (1998) Nitric oxide synthase in rat neuromuscular junctions and in nerve terminals of Torpedo electric worgan: its role as regulator of acetylcholine release. *J. Neurosci. Res*. **51**, 90-102.
32. Rodgers, G. P., Dover, G. J., Uyesaka, N., Noguchi, C. T., Schechter, A. N., & Nienhuis, A. W. (1993) Augmentation by erythropoietin of the fetal-hemoglobin response to hydroxyurea in sickle cell disease. *N. Engl. J. Med*. **328,** 73-80.
33. Sanes J. R. & 20 co-authors (1998) Development of the neuromuscular junction:: genetic analysis in mice. *J. Physiol*. **92,** 167-172.
34. Silvagno, F., Xia, H. H., & Bredt, D. S. (1996) Neuronal nitric-oxide synthase-mu, an alternative spliced isoform expressed in differentiated skeletal muscle. *J. Biol. Chem.* **271,** 11204-11208.
35. Takemitsu, M., Ishiura, S., Koga, R., Kamakura, K., Arahata, K., Nonaka, I., & Sugita, H. (1991) Dystrophin-related protein in fetal and denervated skeletal muscles of normal and mdx mice. *Biochem. Biophys. Res. Comm*. **180,** 1179-1186.
36. Tinsley, J. M., & Davies, K. E. (1993) Utrophin: a potential replacement for dystrophin? *Neuromusc. Disord.* **3,** 537-539.
37. Tinsley, J. M., Blake, D. J., Pearce, M., Knight, A. E., Kendrick-Jones, J., & Davies, K. E. (1993) Dystrophin and related proteins. *Curr. Opin. Genet. Dev.* **3,** 484-490.
38. Tinsley, J. M., Potter, A. C., Phelps, S. R., Fisher, R., Trickett, J. I., & Davies, K. E. (1996) Amelioration of the dystrophic phenotype of mdx mice using a truncated utrophin transgene. *Nature* **384,** 349 -353.
39. Tinsley, J. M., Deconinck, N., Fisher, R., Kahn, D., Phelps, S., Gillis, J. M., & Davies, K. E. (1998) Expression of full-length utrophin prevents muscular dystrophy in mdx mice. *Nat. Med*. **4,** 1441 - 1444.
40. Vater, R., Young, C., Anderson, L. V. B., Lindsay, S., Blake, D. J., Davies K. E., Zuellig, R., & Slater, C. R. (1998) Utrophin mRNA expression in muscle is not restricted to the neuromuscular junction. *Mol. Cell. Neurosci.* **10,** 229 -242.

## Revendications

1. Utilisation d'au moins un donneur de NO ou d'un substrat de la NO synthase sélectionné parmi la L-arginine et ses dérivés dans la préparation d'un médicament pour la réactivation de l'expression de l'utrophine pour le traitement de la myopathie de Duchenne ou de la myopathie de Becker.

2. Utilisation selon la revendication 1, **caractérisé en ce que** ledit donneur de NO ou substrat de la NO synthase est une combinaison de L-arginine et SIN-1.

3. Utilisation selon l'une quelconque des revendications 1 et 2, **caractérisée en ce qu'**elle permet en outre de restaurer la localisation d'utrophine au niveau du sarcolemme.

## Claims

1. Use of at least one NO donor or of a substrate for NO synthase selected from L-arginine and its derivatives, in the preparation of a medicinal product for reactivating the expression of utrophin for the treatment of Duchenne muscular dystrophy or of Becker muscular dystrophy.

2. Use according to Claim 1, **characterized in that** said NO donor or substrate for NO synthase is a combination of L-arginine and SIN-1.

3. Use according to either one of Claims 1 and 2, **characterized in that** it also makes it possible to restore the location of utrophin in the sarcolemma.

## Patentansprüche

1. Verwendung von wenigstens einem NO-Donor oder einem Substrat der NO-Synthase, ausgewählt unter L-Arginin und dessen Derivaten, bei der Herstellung eines Arzneimittels für die Reaktivierung der Expression von Urotrophin für die Behandlung von Duchenne-Muskeldystrophie oder Becker-Muskeldystrophie.

2. Verwendung nach Anspruch 1, **dadurch gekennzeichnet, dass** der NO-Donor oder das Substrat der NO-Synthase eine Kombination von L-Arginin und SIN-1 ist.

3. Verwendung nach einem der Ansprüche 1 und 2, **dadurch gekennzeichnet, dass** sie außerdem erlaubt, die Lokalisierung von Urotrophin auf der Ebene des Sarkolemms wiederherzustellen.
